# EUROPEAN PATENT APPLICATION

(11) **EP 2 612 888 A1**
(43) Date of publication of application: **10.07.2013**
(21) Application number: 12150527.5
(22) Date of filing: 09.01.2012
(51) Int. Cl.: C08L 89/00, C07K 14/78, C08L 89/06, A61L 24/10, C08J 9/28

(54) **A modified collagen**

(71) Applicant: Innocoll Technologies Limited, Athlone (IE)
(72) Inventor: Myers Michael, Ashburn, VA 20148 (US); Dietrich Alexandra, 93342 Saal/Donau (DE)
(74) Representative: O'Connell, Maura

(57) **Abstract**

The present invention relates to a modified collagen obtainable by providing isolated collagen, optionally an isolated collagen dispersion; freezing the isolated collagen; and dehydrating the frozen isolated collagen. The present invention also relates to a method for preparing a modified collagen, the method comprising the steps of providing isolated collagen; freezing the isolated collagen; and dehydrating the frozen collagen. Also disclosed are compositions comprising a modified collagen according to the present invention, uses thereof, and methods for the manufacture thereof.

## Description

### Background to the Invention

Processes for the preparation of collagen-based materials for use in human and veterinary medicine by drying or lyophilizing aqueous collagen dispersions to create membranes or sponges are well known in the art. The use of collagen-based films or membranes as temporary, biodegradable barriers for separating apposing traumatized tissue surfaces following surgery to prevent or reduce the formation of postoperative adhesions is also known.

Typically, the collagen used for subsequent manufacture of the collagen-based materials is first isolated by extraction from mammalian hide or tendon, purified, enzymatically-treated to remove the non-helical telopeptides, partially solubilised with acid, and finally precipitated by increasing the pH to provide an aqueous dispersion of purified, fibrillar collagen. Once isolated, the collagen dispersion may be further processed for the manufacture of collagen-based materials immediately, or is otherwise stored while waiting further processing. For storage convenience at commercial scale, the collagen dispersion is normally concentrated by removal of water using centrifugation to reduce bulk and thereby create a wet mass. The wet mass must be stored frozen to preserve the collagen and prevent bacterial growth. When needed for manufacture of collagen-based materials, the frozen collagen wet mass is typically thawed and redispersed. Whether the isolated collagen is used immediately or frozen and thawed as a wet mass, the collagen dispersion is generally viscous and difficult to process at commercial scale into collagen-based membranes or lyophilized sponges. What is needed is a method to reduce the viscosity of the collagen dispersion without further dilution, since reducing the collagen concentration in the dispersion will only increase the amount of water that must be removed on subsequent drying or lyophilizing, which is both inefficient and costly at commercial scale.

Therefore, the object of the present invention is to modify the isolated collagen in such a way as to reduce the viscosity of the dispersion, but without compromising the properties of the collagen-based materials made thereof. Preferably, a further object of the present invention is to modify the collagen in such a way as to reduce the viscosity of the dispersion and also improve the properties of a collagen membrane made thereof for use as a postoperative adhesion barrier.

These objects are solved according to the present invention by providing a modified collagen that facilitates the efficient manufacture of collagen-based materials at commercial scale and improves the potential effectiveness of those materials in the field of human and veterinary medicine.

### Summary of the Invention

According to a first aspect of the present invention there is provided a modified collagen obtainable by providing isolated collagen, optionally an isolated collagen dispersion; freezing the isolated collagen; and dehydrating the frozen isolated collagen.

By the term "dispersion" is meant a mixture in which collagen particles are dispersed in a fluid, optionally a liquid, further optionally an aqueous, medium. The collagen particles may comprise collagen molecules, or aggregates thereof; which are dispersed in a fluid, optionally a liquid, further optionally an aqueous, medium. Optionally, the collagen particles, which are dispersed in a fluid, optionally a liquid, further optionally an aqueous, medium; have a length (or maximum dimension) of at least one micrometer.

According to a second aspect of the present invention there is provided a method for preparing a modified collagen, the method comprising the steps of:
(a) providing isolated collagen, optionally an isolated collagen dispersion;
(b) freezing the isolated collagen; and
(c) dehydrating the frozen collagen.

Optionally, the providing step comprises the step of removing the fluid, optionally the liquid, further optionally the aqueous, medium; prior to the providing step. Further optionally, the providing step comprises the step of removing at least some of the fluid, optionally the liquid, further optionally the aqueous, medium; prior to the providing step. Still further optionally, the providing step comprises the step of removing at least some of the fluid, optionally the liquid, further optionally the aqueous, medium; prior to the providing step; to provide an isolated collagen dispersion.

Optionally, the providing step comprises the step of removing the fluid, optionally the liquid, further optionally the aqueous, medium; prior to the providing step to provide a dispersion having a concentration of about 3-30%, optionally 3-4%, (w/w) collagen particles.

Optionally, the freezing step comprises freezing to a temperature of about -33°C to about -42°C. Further optionally, the freezing step comprises freezing to a temperature of about -38°C. Still further optionally, the freezing step comprises freezing at a rate of about 0.3°C to about 1.5°C per minute, optionally a rate of about 0.5°C per minute.

Optionally, the dehydrating step comprises removing the aqueous phase. Further optionally, the dehydrating step comprises removing the aqueous phase by reducing the pressure. Still further optionally, the dehydrating step comprises removing the aqueous phase by reducing the pressure to about 0.05 to about 0.5 mbar. Still further optionally, the dehydrating step comprises removing the aqueous phase by applying a vacuum.

Optionally or additionally, the dehydrating step comprises increasing the temperature of the frozen collagen. Further optionally or additionally, the dehydrating step comprises increasing the temperature of the frozen collagen under vacuum. Still further optionally or additionally, the dehydrating step comprises increasing the temperature of the collagen to about +30°C. Still further optionally or additionally, the dehydrating step comprises increasing the temperature of the collagen to about +30°C under vacuum.

Optionally or additionally, the dehydrating step comprises increasing the temperature of the collagen to about +30°C at a rate of about 0.3°C to about 1.5°C per minute, further optionally at a rate of about 0.5°C per minute. Further optionally or additionally, the dehydrating step comprises increasing the temperature of the collagen to about +30°C at a rate of about 0.3°C to about 1.5°C per minute, further optionally at a rate of about 0.5°C per minute, under vacuum.

Optionally, the dehydrating step comprises at least one equilibrating step.

Optionally, the at least one equilibrating step comprises maintaining the temperature at a constant temperature, sufficient to allow the frozen collagen to reach a desired temperature. Further optionally, the at least one equilibrating step comprises maintaining the temperature at a constant temperature for a sufficient period of time to allow the frozen collagen to reach a desired temperature. Still further optionally, the at least one equilibrating step comprises maintaining the temperature at a constant temperature for at least 10mins, optionally at least 20mins, further optionally at least 30mins, still further optionally at least 45mins, still further optionally at least 60mins; to allow the frozen collagen to reach a desired temperature.

Optionally, the at least one equilibrating step is conducted when the temperature is increased to at least -20°C. Optionally or additionally, the at least one equilibrating step is conducted when the temperature is increased to at least -10°C. Optionally or additionally, the at least one equilibrating step is conducted when the temperature is increased to at least 0°C. Optionally or additionally, the at least one equilibrating step is conducted when the temperature is increased to at least +10°C. Optionally or additionally, the at least one equilibrating step is conducted when the temperature is increased to at least +20°C. Optionally or additionally, the at least one equilibrating step is conducted when the temperature is increased to at least +30°C.

Optionally, the dehydrating step comprises six equilibrating steps, each equilibrating step being conducted when the temperature is increased by about 10°C. Further optionally, the dehydrating step comprises six equilibrating steps, each equilibrating step being conducted when the temperature is increased to about -20°C, about -10°C, about 0°C, about +10°C, about +20°C, and about +30°C.

Optionally, the isolated collagen is fibrillar collagen. Further optionally, the isolated collagen is selected from Type I collagen, Type II collagen, Type III collagen, and a mixture thereof. Still further optionally, the isolated collagen is Type I collagen.

Optionally, the method further comprises the step of mechanically degrading the modified collagen. Optionally, the mechanical degrading step comprises milling. Further optionally, the mechanical degrading step is selected from milling, cutting, grinding, granulating, and a mixture thereof.

According to a third aspect of the present invention there is provided a method for isolating collagen, the method comprising the steps of:
(a) providing a collagen source; and
(b) increasing the pH of the collagen source to about 6.5 to about 7.5.

Optionally, the collagen source is a collagen dispersion.

Optionally, the providing step comprises the step of removing the fluid, optionally the liquid, further optionally the aqueous, medium; prior to the providing step. Further optionally, the providing step comprises the step of removing at least some of the fluid, optionally the liquid, further optionally the aqueous, medium; prior to the providing step. Still further optionally, the providing step comprises the step of removing at least some of the fluid, optionally the liquid, further optionally the aqueous, medium; prior to the providing step; to provide an isolated collagen dispersion.

Optionally, the pH of the collagen source, optionally the collagen dispersion, is increased to about 7.5.

Optionally, the collagen source is a fibrous tissue, optionally connective tissue. Further optionally, the collagen source is tendon, optionally animal tendon, further optionally equine or bovine tendon, preferably equine tendon.

Optionally, the method comprises the step of degrading the collagen source prior to the pH-increasing step. Further optionally, the degrading step comprises mechanically degrading the collagen source prior to the pH-increasing step. Optionally or additionally, the degrading step comprises chemically degrading the collagen source prior to the pH-increasing step.

Optionally, the mechanical degrading step comprises milling. Further optionally, the mechanical degrading step is selected from milling, cutting, grinding, granulating, and a mixture thereof. Optionally or additionally, the chemical degrading step comprises contacting the collagen source with an enzyme, optionally a proteolytic enzyme. Optionally, the proteolytic enzyme is selected from chymosin, cathepsin E, and pepsin; preferably pepsin.

Optionally, the chemical degrading step is conducted at a pH of about 2.5.

Optionally, the method further comprises the step of removing contamination from the collagen source. Optionally, the removing step comprises contacting the collagen source with a base, optionally a strong base, further optionally sodium hydroxide, still further optionally an aqueous solution of sodium hydroxide.

Optionally, the method comprises the step of filtering the degraded collagen source, optionally the degraded collagen dispersion, prior to the pH-increasing step.

Optionally, the method comprises the step of concentrating the collagen. Optionally, the concentrating step comprises isolating the collagen. Further optionally, the concentrating step comprises isolating the collagen by centrifugation.

Optionally, the concentrating step comprises the step of removing the fluid, optionally the liquid, further optionally the aqueous, medium; to provide a dispersion having a concentration of about 3-30%, optionally 3-4%, (w/w) collagen particles.

Optionally, the isolated collagen is frozen. Further optionally, the isolated collagen is frozen at less than -20°C. Optionally, the frozen isolated collagen is thawed prior to preparing the modified collagen.

According to a fourth aspect of the present invention, there is provided a composition comprising a modified collagen according to a first aspect of the present invention, or a modified collagen prepared according to a second aspect of the present invention, for use in treating or preventing surgical adhesions.

Optionally, use comprises the administration of the composition at a biological membrane, optionally a biological tissue. Further optionally, use comprises the administration of the composition at a biological membrane, optionally a biological tissue, within a body cavity. Still further optionally, use comprises the administration of the composition at a biological membrane, optionally a biological tissue, within a body cavity such as a peritoneal cavity, a pericardial cavity, a uterine cavity, or a synovial cavity.

Optionally, use comprises the topical administration of the composition at a biological membrane, optionally a biological tissue. Further optionally, use comprises the topical administration of the composition at a biological membrane, optionally a biological tissue, within a body cavity. Still further optionally, use comprises the topical administration of the composition at a biological membrane, optionally a biological tissue, within a body cavity such as a peritoneal cavity, a pericardial cavity, a uterine cavity, or a synovial cavity.

According to a fifth aspect of the present invention there is provided a method for the manufacture of a composition comprising a modified collagen according to a first aspect of the present invention or a modified collagen prepared according to a second aspect of the present invention, the method comprising the steps of:
(a) providing a modified collagen;
(b) preparing an aqueous dispersion of the modified collagen;
(c) degrading the aqueous dispersion; and
(d) dehydrating the aqueous dispersion.

Optionally, the preparing step comprises adding heated water, optionally heated purified water, to the modified collagen. Optionally, the water, optionally the purified water is heated to about 35 to about 42°C prior to adding to the modified collagen.

Optionally, the preparing step is conducted at a pH of about 4.0.

Optionally, the degrading step comprises mechanically degrading the aqueous dispersion. Optionally, the mechanical degrading step comprises shear mixing.

Optionally, the composition comprises modified collagen in an amount of about 0.4% to 1.5% (w/w).

Optionally, the composition has a pH of about 4.0.

Optionally, the dehydrating step comprises removing liquid from the aqueous dispersion such that the composition comprises liquid in an amount of less than 30%, optionally less than 20%, further optionally less than 15% (w/w) of the aqueous dispersion. Further optionally, the dehydrating step comprises removing liquid from the aqueous dispersion such that the composition comprises liquid in an amount of less than 13%, preferably less than 12%, (w/w) of the aqueous dispersion.

Optionally, the dehydrating step comprises removing liquid from the aqueous dispersion using a convective drying cabinet.

According to a sixth aspect of the present invention there is provided a drug delivery composition obtainable by providing isolated collagen, optionally an isolated collagen dispersion; freezing the isolated collagen; and dehydrating the frozen isolated collagen.

According to a seventh aspect of the present invention, there is provided a method of preparing a drug delivery composition for sustained drug release, the method comprising the steps of:
(a) providing isolated collagen, optionally an isolated collagen dispersion;
(b) freezing the isolated collagen; and
(c) dehydrating the frozen collagen.

Optionally, the method further comprises the step of providing a drug, optionally a drug solution, to which the dehydrated frozen collagen is added, or which is added to the dehydrated frozen collagen. Optionally, the drug is an aqueous drug solution. Further optionally, the drug is an aqueous drug solution comprising an acid, optionally acetic acid.

Optionally, the method further comprises the step of mixing, optionally homogenizing, the drug-, optionally drug solution-, containing drug delivery composition.

Optionally, the method further comprises the step of lyophilizing and/or dehydrating, the drug-, optionally drug solution-, containing drug delivery composition.

### Brief Description of the Drawings

Embodiments of the present invention will now be described with reference to the following nonlimiting examples and the accompanying drawings wherein the error bars represent standard deviations, in which:

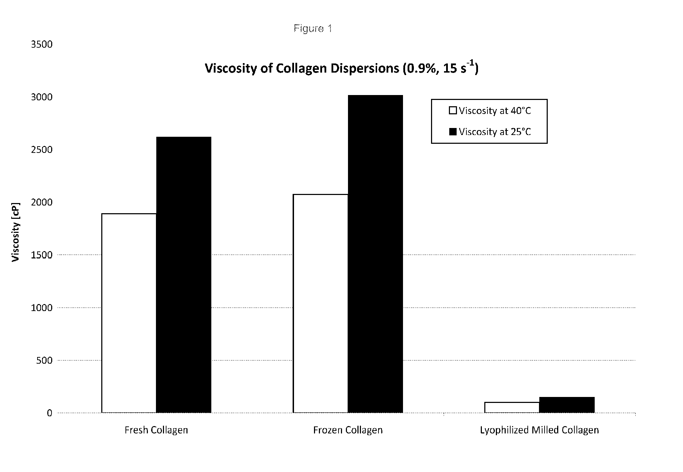

**Figure 1** is a graph illustrating the viscosity characteristic of compositions prepared from fresh collagen, frozen collagen, and a modified collagen according to a first aspect of the present invention (lyophilised milled collagen);
**Figure 2A** is a graph illustrating the water uptake characteristic of compositions prepared from fresh collagen, frozen collagen, and a modified collagen according to a first aspect of the present invention (lyophilised milled collagen);
**Figure 2B** is a graph illustrating the swelling characteristic of compositions comprising fresh collagen, frozen collagen, and a modified collagen according to a first aspect of the present invention (lyophilised milled collagen);
**Figure 3A** is a graph illustrating the dissolution characteristic of gentamicin-containing compositions prepared from frozen collagen and a modified collagen according to a first aspect of the present invention (lyophilised milled collagen); and
**Figure 3B** is a graph illustrating the dissolution characteristic of bupivacaine-containing compositions prepared from frozen collagen and a modified collagen according to a first aspect of the present invention (lyophilised milled collagen).

### Examples

### Example 1 - Collagen Isolation

Collagen can be isolated from a number of sources, for example, animal hides and animal tendons. In a preferred embodiment, the collagen is isolated from animal tendon, for example equine or bovine tendon; although any known source of collagen, including fibrous tissue, optionally connective tissue, may be used and selected by one skilled in the art. Preferably, the collagen is isolated from equine tendon. In the method of isolation, equine tendons were milled to degrade the collagen source. The milled equine tendons were treated with a number of reagents, including 1 N sodium hydroxide (NaOH) to remove microbiological contamination such as prions at the beginning of the process. Treatment steps with hydrogen peroxide and washing steps at different pH values were conducted, followed by a milling step, which was used to increase the surface for the next treatment step. The molecular weight of the collagen source was additionally reduced by treatment with the proteolytic enzyme pepsin at an approximate pH of 2.5. The pH was adjusted using an aqueous solution of 1 N HCl. The pepsin was used to degrade contaminating serum components such as equine serum albumin (ESA) and resulted in the detachment of non-helical portions of the collagen molecule (telopeptides). During this process, the collagen material was also partially solubilised in the acidic medium. After filtration, the pH level was increased from 2.5 to 7.5 by addition of 1 N sodium hydroxide (NaOH). This pH adjustment resulted in precipitation of the fibrillar collagen out of solution, which was then concentrated by means of centrifugation to provide a collagen dispersion having a concentration of about 3-30% (w/w). The resulting material was designated fresh collagen. The fresh collagen can be processed in several ways.

The fresh collagen can be packaged in suitable portions and frozen to -20°C to be stored in a freezer until required for use. The resulting material was designated frozen collagen. The frozen collagen is thawed prior to use in the same manner as fresh collagen.

Alternatively, frozen collagen can be freeze-dried (lyophilised), and optionally subsequently milled. The resulting material was designated lyophilised milled collagen. For this purpose, frozen collagen was manually distributed onto a flat surface, for example a polystyrene mould, having a layer thickness of between about 5mm and about 10mm. The collagen-filled moulds were transferred onto the shelves of a commercially available freeze dryer (Christ Epsilon) and frozen to a temperature of about -38°C with a ramp rate between 0.3°C and 1.5°C. After an equilibration period of approximately 30 minutes vacuum was initiated and the shelf temperature was sequentially increased from about ―38°C to about +30°C at a rate of about 0.5°C per minute. The combination of vacuum and sequentially increasing the shelf temperature from about ―38°C to about +30°C facilitated sublimation of the ice from the frozen collagen up until the collagen reached a temperature of 0°C. To ensure that the temperature of the collagen increased uniformly, at least one equilibrating step was conducted, in which the shelf temperature was maintained at a constant desired temperature for approximately 30mins, or until the collagen reached the desired temperature. For example, an equilibrating step was conducted every 10°C between the temperatures of -20°C and +30°C to ensure that the temperature of the collagen increased uniformly. The equilibrating step, for example at -20°C comprised maintaining the shelf temperature at a constant temperature of -20°C for about 30mins. Once the ice had been removed by sublimation, and the collagen reached a temperature of 0°C, the residual water content was further reduced by continuing to sequentially increase the shelf temperature to about +30°C at a rate of about 0.5°C per minute. The lyophilised collagen was then milled using a commercially available cutting mill (Rotoplex, Hosokawa Alpine). The lyophilised milled collagen was stored in polyethylene containers (bags) until required for use. The resulting material was designated modified collagen (lyophilized milled collagen; LMC).

### Example 2 - Compounding Process and Equipment

An aqueous modified collagen dispersion was prepared in a stainless steel vessel using pre-heated (35 ― 42°C) purified water, which was adjusted to pH 4.0 ± 0.2. High shear mixing was required to break up the modified collagen mass and expose the collagen fibres to the acidic medium. The high shear mixer (homogeniser) comprised a rotor/stator head that is designed to create high shear forces by pulling the modified collagen through the rotating homogeniser head and forcing the modified collagen against the proximal stationary stator head. It is this design that provided the high shear forces required to separate the fibrous collagen mass at the beginning of the aqueous dispersion preparation. However, other comparable mixing equipment may also be used; and can be selected by one skilled in the art. For example, an IKA Ultra-Turrax mixer may be used at a high speed for about 2 to about 5 minutes.

If required, although not essential, the resulting aqueous dispersion can be filtered and degassed, for example by using 250 micron filters and a suitable means of degassing, for example ultrasonication.

The collagen concentration in the final aqueous dispersion can be in the range of 0.4% to 1.5% and the pH can be in the range of 4.0 ± 0.2. The final aqueous dispersion can be subsequently transferred to a closed jacketed stainless steel vessel, optionally where the jacket temperature is maintained at 37°C and the aqueous dispersion is slowly agitated using a low shear setting.

The dispersion was filled into, for example 10 x 10 cm, blister trays or lyophilisation moulds using, for example, a positive displacement pump. The pump can be a valve-less pump, optionally having ceramic pistons. Alternatively, a peristaltic pump could also be used. The fill weight was adjusted based on the collagen content of the aqueous dispersion to achieve the target collagen content per area, for example about 0.1 to about 10.0 mg/cm², optionally about 4 mg/cm². Upon completion of the filling process, the filled blisters or moulds were placed into a convective drying cabinet. A commercially available drying cabinet (LabAir; Bleymehl) at 31°C was utilized for this drying process. The drying step can typically require between 1 and 3 days to remove the excess water, which results in the finished composition, for example membrane, being retained in the blisters or moulds.

Following completion of the drying process, the blisters or moulds were removed from the drying cabinet. The resulting composition, for example membrane, was cut to the desired size, for example using a pneumatic dye. The packaging process was a two-step process comprising introduction to an inner and outer pouch packaging (ethylene oxide; EO type; PMS MEDICAL LTD) followed by pneumatic heat sealing. One side of the outer pouch comprised a transparent polyester or low-density polyethylene (LDPE) foil laminate with a high-density polyethylene (HDPE) strip seal. The other side was an opaque polyester or LDPE laminate. Other outer pouch packaging material can be used, including aluminum oxide coated polyethylene materials or, if E-beam radiation is used for sterilization, an aluminum outer pouch can be used. The pneumatic heat sealer facilitated the formation of a continuous seal at the open end of the pouch. The top part of the pouch included two holes or strips lined with a high-density polyethylene (HDPE) strip seal. These openings / windows were specifically designed for the EO gas sterilisation process and were gas permeable only. The permeability of the window facilitated permeation of the EO gas during the terminal EO sterilization process. Following sterilization and ventilation, the outer pouch was resealed below the gas permeable openings / windows, and this gas permeable (top) portion was then removed from the pouch. This resulted in a fully sealed outer pouch containing a terminally sterilized finished composition, for example membrane.

Ethylene Oxide (EO; C₂H₄O) is a gas that, at appropriate operating temperatures, sterilises via the action as a powerful alkylating agent. Under the correct conditions, cellular constituents of organisms such as nucleic acid complexes, functional proteins, and enzymes will react with ethylene oxide, causing the addition of alkyl groups. As a result of the alkylation, cell reproduction is prevented and cell death ensues. The sterilizer used in the present Examples was a DMB 15009 VD (DMB Apparatebau GmbH, Germany). A mixture of EO / CO₂ at a ratio of 15:85 was used as the sterilization gas over a period of 6 hours at 4 bar pressure. For successful completion of this process, the product needs to contain a moisture level of not less than 9%, which can be achieved by holding it in an area under controlled environmental conditions. Following the EO sterilization process, the product was ventilated for a minimum of 3 to 4 weeks to reduce the level of remaining ethylene oxide gas and any residues from the composition, for example membrane, and packaging materials.

### Example 3 - Characterisation

All compositions (membranes) were prepared from a 0.6% dispersion using the method described herein above. All tests on the collagen dispersion were conducted within 1 day after compounding; and all characterisation experiments with the membranes were performed within 1 month after membrane manufacture using unsterilised membranes.

### Dispersion Viscosity

The viscosity values of each of the fresh collagen, frozen collagen, and lyophilised milled collagen were measured using a Brookfield viscometer (Digital Rheometer DV-III+ with associated TC-501 Circulating Bath). The viscosity values were measured at a constant shear rate (15 revolutions per second) and over a temperature range from 25 to 40°C at 5°C increments. 60 measurements per temperature were averaged to obtain reliable results.

The dispersion viscosity depends on the temperature and decreases when heating up the dispersion. The viscosity profiles of fresh and frozen collagen are comparable over the temperature range tested. The lyophilised milled collagen showed significantly lower viscosity at all investigated temperatures compared to the fresh collagen and the frozen collagen.

The difference in viscosity is an advantage for processing of the membranes. Collagen with lower viscosity can be more easily degassed, and filled or casted; and the drying time is also reduced as collagens having higher concentrations can be processed.

### Water Uptake and Swelling

Three rectangular samples (1.5 x 4 cm in size) were cut from 5 membranes prepared from each of the fresh collagen, frozen collagen, and lyophilised milled collagen. Each of these samples was soaked in WFI (water for injection) for 10 minutes, and analysed regarding water uptake (wet weight ― dry weight) and swelling (wet thickness ― dry thickness). The sample thickness was measured using a Mitutoyo Micrometer IP54.

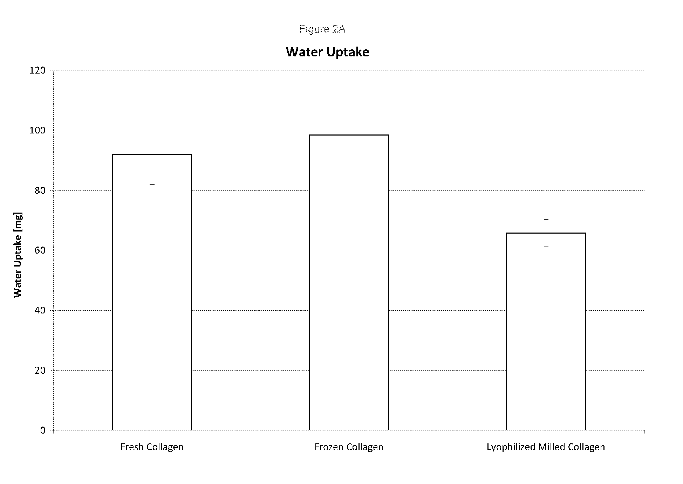

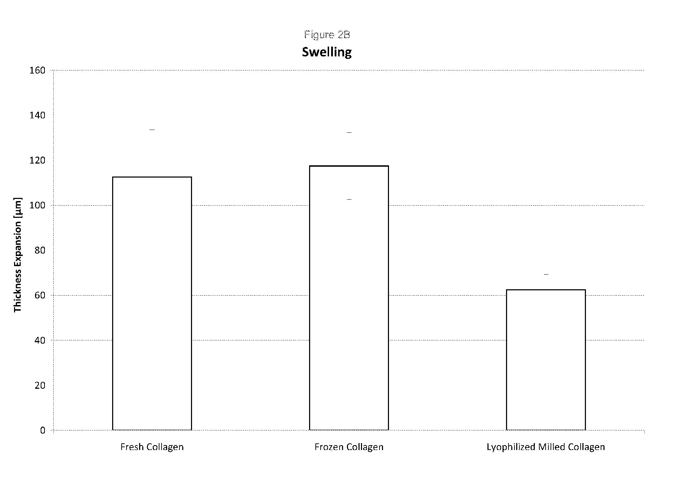

Membranes prepared from lyophilised milled collagen showed lower water uptake and swelling than membranes prepared from fresh collagen and from frozen collagen (see Figure 2a and 2b). The variability of results was substantially lower for membranes prepared from lyophilised milled collagen than for the membranes prepared from fresh collagen and from frozen collagen.

The reduced swelling characteristics of membranes prepared from lyophilised milled collagen is advantageous as the membranes may be implanted into restricted anatomical spaces with a lower risk of pressurising and potentially damaging vital organs. Thus, for use in treating or preventing surgical adhesions, membranes prepared from the modified collagen may be used in a greater variety of anatomical geometries and surgical procedures.

### Degradation with Collagenase

Degradation studies were conducted using 4 to 5 membranes per batch of each of the fresh collagen, frozen collagen, and lyophilised milled collagen. One membrane (4.5 x 4.5cm in size) was placed into a beaker and covered with 15mL of 0.2N Phosphate buffer (pH 7.4 with CaCl₂). Collagenase (Collagenase Type IA-S, sterile, 50 mg, SIGMA, REF C5894) was reconstituted with 5mL of WFI, and 0.5mL of the resulting solution was added to the mixture. The solution in the beaker was agitated using a shaking water bath (Julabo SW 22) at 37°C (120 rpm) for 60 minutes. The degradation was documented by taking photographs of the samples every 5 minutes. Results are shown in Table 1. Membranes prepared from lyophilised milled collagen degraded the fastest with no residue, while membranes prepared from fresh collagen and frozen collagen degraded considerably slower, and left behind small fibre agglomerates.

**Table 1: Degradation of equine collagen membranes in presence or Collagenase**

| | **Fresh Collagen** | **Frozen Wet Collagen** | **Lyophilized Milled Collagen** |
|---|---|---|---|
| Dissolution [min] | 50 | 50 | 25 |

A composition for use in treating or preventing surgical adhesions, for example a membrane for use as an adhesion barrier, needs to stay intact for a certain time in order to effectively inhibit adhesion. Prolonged presence of the membrane could lead to increased risk of infections, given that collagen is known to be a medium for bacterial growth. These *in vitro* experiments demonstrate that the membranes prepared from lyophilised milled collagen degrade faster than membranes prepared from fresh collagen and frozen collagen, suggesting that this effect will also be true for the *in vivo* behaviour. Accordingly, a composition comprising a modified collagen according to a first aspect of the present invention, or a modified collagen prepared according to a second aspect of the present invention, for use in treating surgical adhesions, can reduce the probability of infections as an adverse effect of the use of the adhesion barrier.

Taken together, the examples provided herein demonstrate that a composition comprising a modified collagen according to a first aspect of the present invention, or a modified collagen prepared according to a second aspect of the present invention - for example, membranes prepared from lyophilised milled collagen - exhibit significantly altered properties compared to membranes made from fresh collagen or frozen collagen; and can be particularly useful in preventing or treating surgical adhesions.

### Example 4 ― Dissolution

### Preparation of compositions comprising gentamicin

Compositions (sponges) containing gentamicin sulfate (Fujian Fukang Pharmaceutical Co. Ltd, China) were prepared for dissolution testing from a 1.6% w/w collagen dispersion using a modified version of the method described herein above. Each sponge measured 2.5 x 2.5 x 0.5 cm and contained 50mg of collagen and 50mg of gentamicin sulfate. In short, gentamicin sulfate (1.6% w/w) and 1 N Acetic Acid were added to water for injection (WFI), and stirred until a clear solution resulted. Collagen (1.6% w/w) was added to the solution, either as frozen collagen (thawed directly prior to production) or as a modified collagen according to the present invention (Lyophilized Milled Collagen; LMC). The mixture was homogenized using a commercially available high shear mixer (Ultraturrax, IKA, Germany) for 1 to 5 minutes at a temperature between 38 and 42°C until a homogeneous viscous dispersion was obtained. The dispersion was filtered through a 250µm mesh and stirred for approximately 30 minutes. Aliquots of the dispersion were filled into blisters and placed onto the shelves of a suitable freeze dryer and lyophilized. The blisters filled with dispersion were transferred onto the shelves of a commercially available freeze dryer and frozen to a temperature of about -38°C with a ramp rate between 0.3°C and 1.5°C. After an equilibration period of approximately 30 to 60 minutes, vacuum was initiated and the shelf temperature was sequentially increased from about ―38°C to about +30°C at a rate of about 0.5°C per minute. The combination of vacuum and sequentially increasing the shelf temperature from about ―38°C to about +30°C facilitated sublimation of the ice from the frozen dispersion up until the product reached a temperature of 0°C. To ensure that the temperature of the collagen increased uniformly, at least one equilibrating step was conducted, in which the shelf temperature was maintained at a constant desired temperature for at least 30mins, or until the collagen reached the desired temperature. The sponge-like porous composition was removed from the blister cavities and packed in pouches as described in Example 2 herein above.

### Preparation of compositions comprising bupivacaine

Compositions (sponges) containing bupivacaine HCl were produced for dissolution testing according to a similar method as described above. Each sponge measured 5 x 5 x 0.5 cm and contained 75mg of collagen and 100mg of bupivacaine HCl. In short, 1 N Acetic Acid was added to WFI and briefly mixed. Collagen (0.6% w/w; either frozen collagen or Lyophilized Milled Collagen) was added to the solution. The mixture was homogenized using a commercially available high shear mixer (Ultraturrax, IKA, Germany) for 1 to 5 minutes at a temperature between 38 and 42°C until a homogeneous viscous dispersion was obtained. The dispersion was filtered through a 250µm mesh. Bupivacaine HCl (0.8% w/w) was dissolved in a small amount of WFI and added to the collagen dispersion. The mixture was stirred for approximately 30 minutes. Aliquots of the dispersion were filled into moulds and transferred onto the shelves of a commercially available freeze dryer and frozen to a temperature of about -38°C with a ramp rate between 0.3°C and 1.5°C. After an equilibration period of approximately 30 to 60 minutes vacuum was initiated and the shelf temperature was sequentially increased from about ―38°C to about +30°C at a rate of about 0.5°C per minute. The combination of vacuum and sequentially increasing the shelf temperature from about ―38°C to about +30°C facilitated sublimation of the ice from the frozen dispersion up until the product reached a temperature of 0°C. To ensure that the temperature of the collagen increased uniformly, at least one equilibrating step was conducted, in which the shelf temperature was maintained at a constant desired temperature for at least 30mins, or until the collagen reached the desired temperature. The sponge-like porous compositions were removed from the moulds and packed in pouches, as described in Example 2 herein above.

### Gentamicin dissolution studies

The dissolution properties of compositions (sponges) containing gentamicin sulfate were analyzed in duplicate using a Dissolution Apparatus Type II (Distek Inc., USA), according to the manufacturer's instructions. To prevent the sponges from floating, they were placed into custom-made stainless steel sinkers. The weighted sponges were immersed in 500mL of PBS Buffer (phosphate buffered saline, pH 7.4, bath temperature 37°C) and stirred at 50rpm for 24 hours. 4.0mL sample was removed after 5, 10, 30, 45, 60, 120, 180, 240 and 1440 minutes. The samples were subjected to a chemical derivatisation reaction with Phthalaldehyde (4 mL sample + 1.6 mL of a solution comprising 1% Phthalaldehyde + 4.4 mL methanol) at 60°C for 15 min (dilution 4/10). The resulting solutions were filtered and analyzed in a HPLC system (Shimadzu Corp., Japan), according to the manufacturer's instructions. A RP-18 HPLC column and a mobile phase comprising WFI, methanol, acetic acid and Na-1-heptanesulfonate with a flow rate of 0.5 mL/min was used. The gentamicin peaks C1, C2 and C2a at 330 nm were integrated, and the gentamicin concentration was calculated from the area under the curve of the samples and from a reference standard that was subjected to identical sample preparation.

### Bupivacaine dissolution studies

The dissolution properties of compositions (sponges) containing bupivacaine HCl were analyzed in duplicate using a Dissolution apparatus Type II (Distek Inc., USA), as described above. To prevent the sponges from floating, they were placed into custom-made stainless steel sinkers. In short, the weighted sponges were immersed in 500 mL of PBS Buffer (phosphate buffered saline, pH 6.8, bath temperature 37°C) and stirred with 50 rpm for 24 hours. 4.0 mL sample was removed after 5, 10, 30, 45, 60, 120, 180, 240 and 1440 minutes. The samples were diluted 1:1 with PBS buffer, filtered and analyzed in a HPLC system (Shimadzu Corp., Japan). A RP-18 HPLC column and a mobile phase comprising phosphate buffer pH 4.5 and acetonitrile with a flow rate of 0.5 mL/min was used. The bupivacaine peak at 230 nm was integrated, and the bupivacaine concentration was calculated from the area under the curve of the samples and from a reference standard.

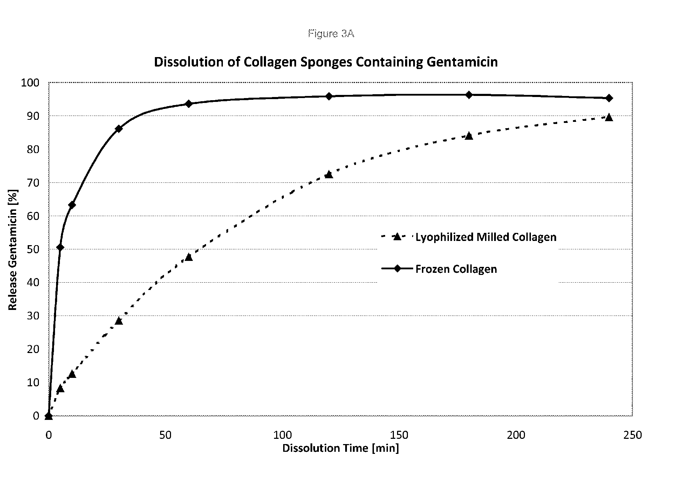

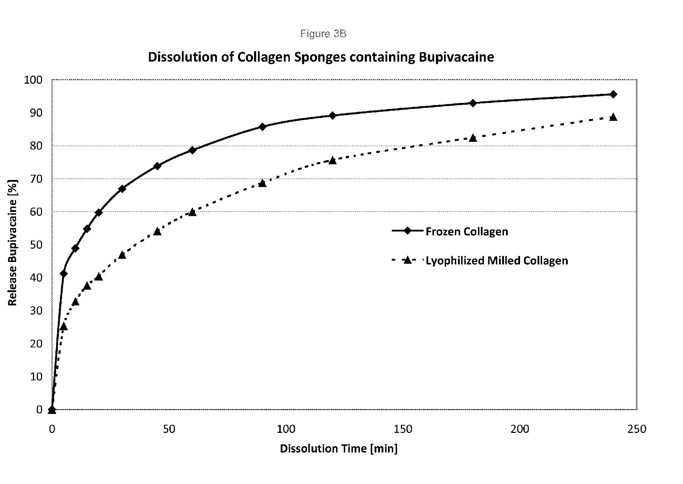

The results of the dissolution studies are illustrated in Figures 3A and 3B.

The results of these dissolution studies demonstrate that a modified collagen according to the present invention provides a drug delivery composition, wherein the rate of release of biologically active substances from the collagen-based composition is reduced relative to those compositions made from isolated collagen without modification, thereby providing a drug delivery composition having a more sustained action of drug release. This extended release can be beneficial for collagen-based products containing active pharmaceutical ingredients (API) with good solubility in water. A retardation of release kinetics for this combination is otherwise difficult to achieve without chemical cross-linking of the drug delivery composition. Both in topical and implant administration, the extended release of the ingredient from the drug delivery composition can lead to longer therapeutic action and improved local efficacy.

## Claims

1. A modified collagen obtainable by providing isolated collagen; freezing the isolated collagen; and dehydrating the frozen isolated collagen.

2. A method for preparing a modified collagen, the method comprising the steps of:
(a) providing isolated collagen;
(b) freezing the isolated collagen; and
(c) dehydrating the frozen collagen.

3. A modified collagen according to Claim 1 or a method according to Claim 2, wherein the providing step comprises providing a dispersion having a concentration of about 3-30% (w/w) collagen particles.

4. A modified collagen according to Claim 1 or 3 or a method according to Claim 2 or 3, wherein the freezing step comprises freezing to a temperature of about -33°C to about - 42°C.

5. A modified collagen or a method according to Claim 4, wherein the freezing step comprises freezing at a rate of about 0.3°C to about 1.5°C per minute.

6. A modified collagen according to any one of Claims 1 and 3-5 or a method according to any one of Claims 2-5, wherein the dehydrating step comprises removing the aqueous phase by reducing the pressure.

7. A modified collagen according to any one of Claims 1 and 3-6 or a method according to any one of Claims 2-6, wherein the dehydrating step comprises increasing the temperature of the collagen to about +30°C.

8. A modified collagen according to any one of Claims 1 and 3-7 or a method according to any one of Claims 2-7, wherein the dehydrating step comprises increasing the temperature of the collagen to about +30°C at a rate of about 0.3°C to about 1.5°C per minute.

9. A modified collagen according to any one of Claims 1 and 3-8 or a method according to any one of Claims 2-8, wherein dehydrating step comprises at least one equilibrating step of maintaining the temperature at a constant temperature for at least 10mins to allow the frozen collagen to reach a desired temperature, wherein each equilibrating step is conducted when the temperature is increased by about 10°C.

10. A method for isolating collagen, the method comprising the steps of:
(a) providing a collagen source; and
(b) increasing the pH of the collagen source to about 6.5 to about 7.5.

11. A composition comprising a modified collagen according to any one of Claims 1 and 3-9 or a modified collagen prepared according to any one of Claims 2-9 for use in treating or preventing surgical adhesions.

12. A composition comprising a modified collagen for use according to Claim 11, wherein use comprises the administration of the composition at a biological membrane.

13. A method for the manufacture of a composition comprising a modified collagen, the method comprising the steps of:
(a) providing a modified collagen;
(b) preparing an aqueous dispersion of the modified collagen;
(c) degrading the aqueous dispersion; and
(d) dehydrating the aqueous dispersion.

14. A method according to Claim 13, wherein the preparing step comprises adding water heated to about 35°C to about 42°C to the modified collagen.

15. A method according to Claim 13 or 14, wherein the dehydrating step comprises removing liquid from the aqueous dispersion such that the composition comprises liquid in an amount of less than 30% (w/w) of the aqueous dispersion.
